# EUROPEAN PATENT SPECIFICATION

(11) **EP 1 601 355 B1**
(45) Date of publication and mention of the grant of the patent: **01.08.2007**
(21) Application number: 04716242.5
(22) Date of filing: 02.03.2004
(51) Int. Cl.: G01N 33/68

(54) **DIAGNOSTIC METHOD FOR PREVISION OF KIDNEY TRANSPLANTATION REJECTION**
DIAGNOSTISCHES VERFAHREN ZUR VORHERSAGE VON NIERENTRANSPLANTATIONS-ABSTOSSUNG
MÉTHODE DIAGNOSTIQUE POUR LA PRÉVISION D'UN REJET DE GREFFE DE REIN

(30) Priority: 06.03.2003 IT FI20030058
(43) Date of publication of application: 07.12.2005
(60) Divisional application: 07109545.9
(73) Proprietor: Universita' Degli Studi di Firenze, 50121 Firenze (IT)
(72) Inventor: GALLI, Andrea, I-50144 Firenze (IT); ROMAGNANI, Paola, I-50139 Firenze (IT); SERIO, Mario, I-50012 Bagno a Ripoli (IT); SURRENTI, Calogero, I-50012 Bagno a Ripoli (IT)
(74) Representative: Gervasi, Gemma
(86) International application number: PCT/EP2004/002069
(87) International publication number: WO 2004/078175

(56) References cited:
- EP-A- 0 953 355
- WO-A-01/62238
- WO-A-02/080956
- WO-A-03/098185
- US-A1- 2001 036 631
- US-A1- 2002 018 776
- US-A1- 2002 019 344
- US-B1- 6 369 098
- OKAI TAKASHI ET AL: "Beneficial hepatic effect of troglitazone in a patient with antimitochondrial antibody-negative primary biliary cirrhosis." THE AMERICAN JOURNAL OF GASTROENTEROLOGY. JAN 2002, vol. 97, no. 1, January 2002 (2002-01), pages 209-210, XP001193329 ISSN: 0002-9270
- GOSSET PHILIPPE ET AL: "Peroxisome proliferator-activated receptor gamma activators affect the maturation of human monocyte-derived dendritic cells" EUROPEAN JOURNAL OF IMMUNOLOGY, vol. 31, no. 10, October 2001 (2001-10), pages 2857-2865, XP001193843 ISSN: 0014-2980
- NISHIOJI K ET AL: "Increase of chemokine interferon - inducible protein - 10 ( IP - 10 ) in the serum of patients with autoimmune liver diseases and increase of its mRNA expression in hepatocytes." CLINICAL AND EXPERIMENTAL IMMUNOLOGY, (2001 FEB) 123 (2) 271-9. JOURNAL CODE: 0057202. ISSN: 0009-9104., February 2001 (2001-02), XP001193770
- KRAFT MATTHIAS ET AL: "PPAR-gamma ligands potently inhibit CXC-chemokine secretion in colonic epithelial cell lines" GASTROENTEROLOGY, vol. 120, no. 5 Supplement 1, April 2001 (2001-04), page A.319, XP008032020 & 102ND ANNUAL MEETING OF THE AMERICAN GASTROENTEROLOGICAL ASSOCIATION AND DIGESTIVE DISEASE WEEK; ATLANTA, GEORGIA, USA; MAY 20-23, 2001 ISSN: 0016-5085
- TRAUNER M. ET AL: "Cholestatic syndromes." CURRENT OPINION IN GASTROENTEROLOGY, 19/3 (216-231). REFS: 273 ISSN: 0267-1379 CODEN: COGAEK, 2003, XP008031758
- BERTONI E ET AL: "High Pre-Transplant Serum Levels of CXCL10 Predict Early Renal Allograft Failure" TRANSPLANTATIONSMEDIZIN: ORGAN DER DEUTSCHEN TRANSPLANTATIONSGESELLSCHAFT 2003 GERMANY, vol. 15, no. 2, 2003, pages 91-96, XP008031756 ISSN: 0946-9648
- ROMAGNANI P ET AL: "High expression of chemokines Interferon-gamma Inducible Protein of 10 kDa (IP-10), Monokine induced by Interferon-gamma (Mig) and of their receptor (CXCR3) in different renal diseases" JN JOURNAL OF NEPHROLOGY, vol. 13, no. 6, November 2000 (2000-11), page 453, XP001193776 & 41ST CONGRESS OF THE ITALIAN SOCIETY OF NEPHROLOGY; TAORMINA, ITALY; JUNE 14-JULY 17, 2000 ISSN: 1121-8428
- ROTONDI MARIO ET AL: "High pretransplant serum levels of CXCL10 / IP 10 are related to increased risk of renal allograft failure ." AMERICAN JOURNAL OF TRANSPLANTATION : OFFICIAL JOURNAL OF THE AMERICAN SOCIETY OF TRANSPLANTATION AND THE AMERICAN SOCIETY OF TRANSPLANT SURGEONS, (2004 SEP) 4 (9) 1466-74. JOURNAL CODE: 100968638. ISSN: 1600-6135., September 2004 (2004-09), XP001199657

## Description

### Field of the invention

The present invention is connected to an in vitro diagnostic method for prevision of kidney transplantation rejection.

### State of art

The parameter which today can be used to modulate the immuosuppressive therapy is the PRA (panel of reactive antibodies).

US 2002/018776 and US 2001/036631 describe the use of IP- 10 level after renal transplantation as a marker for renal transplant rejection

In Bertoni et al. "High pre-transplant serum levels of CXCL10 predict early renal allograft failure" Transplantationmedizin: Organ der Deutschen Transplantation Gesellschaft 2003 Germany, vol. 15, no. 2, 2003, pp. 91-96 it is reported that high pre-transplant serum CXCL10 levels are a clinically useful marker for the development of rejection and renal transplant failure, this document was published after October 9, 2003, i.e. after the priority date of the present application.

### Description of the figures

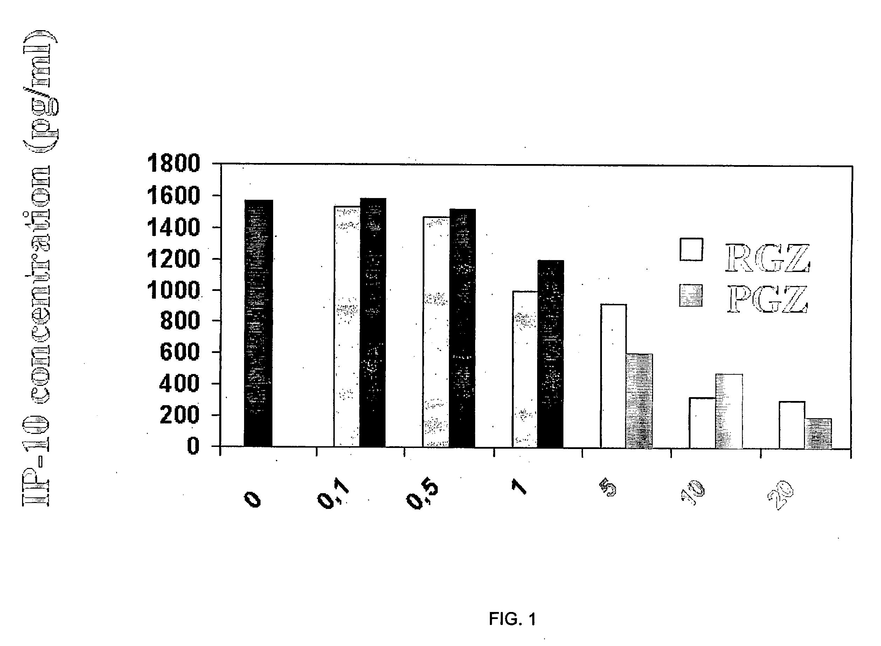

Fig. 1 shows the decreasing production of IP-10 in primary cultures of stellate cells treated with Rosiglitazone and Pioglitazone

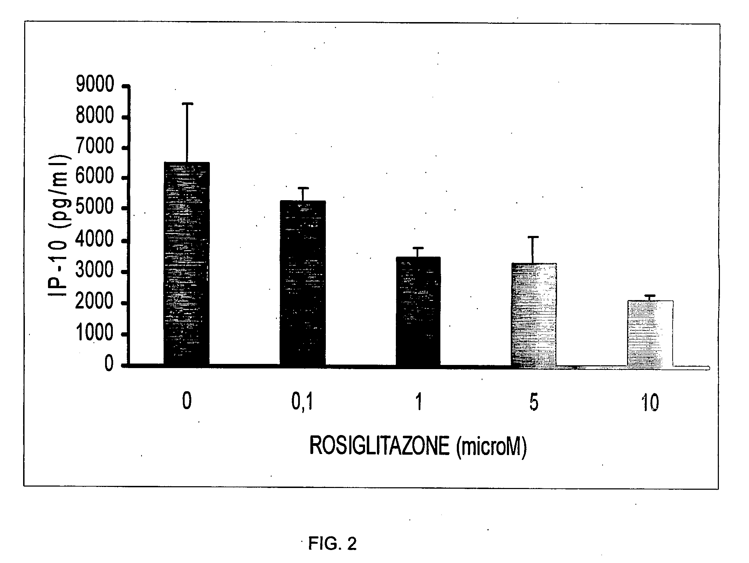

Fig. 2 shows the suppression of the IP-10 levels by treatment of mesangial cells with increasing doses of Rosiglitazone;

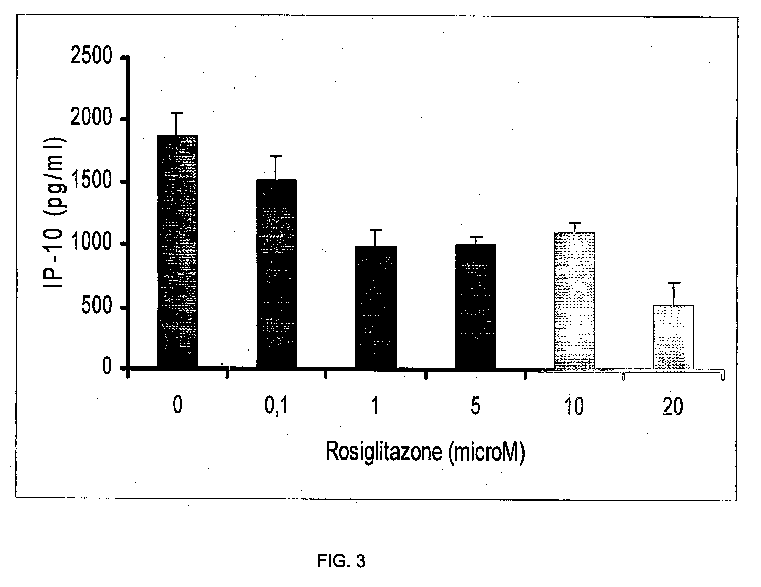

Fig. 3 shows the suppressive effects of Rosiglitazone on the IP-10 levels induced by interferon-γ in epithelial cells treated with increasing doses of Rosiglitazone.

In all the 3 figures the first column (0) indicates the IP-10 level after stimulation with INF-γ and TNF-α in the absence of TzDs.

### Detailed description of the invention

An unsuspected field of application of interferon γ inducible chemokines is the possibility of their use as serum marker for prevision of immune susceptibility of a patient who undergoes to organ transplantation.

We found that the level of IP-10 (CXCL-10) in transplanted patients allows the prevision of allographt rejection with high precision.

The IP-10 chemokine in fact plays an important role in the pathogenesis of acute and chronic rejection of allographt, as demonstrated by animal models.

IP-10 plays a double biological role: it stimulates the migration of lymphocytes, macrophages, dendritic cells and other immunocompetent cells and regulates the vascular physiopathology by induction of mesangial expansion and inhibition of endothelial growth. These biological functions play a fundamental role in acute and chronic allographt rejection and represents together the major cause of morbidity and of transplanted organ loss.

On the basis of the above mentioned experimental evidences, we based our hypothesis that an elevated pre-transplantation level of circulating IP-10 (due to chronic inflammatory stimolous by dyalis and uremia) can induce increased risk of allographt rejection. Therefore, we measured serum CXCL-10 levels before operation in 300 subjects undergoing kidney transplantation, then followed since 5 years from surgical intervention. The healthy controls were 50. In normal subjects the IP-10 levels were 84.4 ± 29.9 pg/ml, while the levels of transplanted subjects were 137.6 ± 123.2 pg/ml. IP-10 pre- transplantation levels were higher in the subjects who lost the allographt (130+ 116 pg/ml in non losers versus 200 ± 163 pg/ml in losers). The subjects who lost the kidney during the first year had pre-transplantation levels higher (211 ± 165.1 vs 130.6 ± 116 pg/ml). The survival curves according Kaplan-Meier, calculated in 300 patients divided in four groups (centiles) on the basis of IP-10 pre-transplantation levels, showed a progressive reduction of transplanted organ survival at 5 years according IP-10 values (97.3%, 94.0%, 93.3%, 85.3% of survival< 0.05 in all groups; p< 0.01 in the group 4 versus 1).

Between the group with the highest pre-transplantation levels of IP-10 and that with the lowest pre-transplantation levels a great difference of allographt loss has been observed (14.7% vs 2.7%, p<0,05). These differences were due to a major frequency of rejections in people with the highest IP-10 pre-transplantation levels.

On these data is based the idea that the pre-transplantation levels of IP-10 can recognize the people with higher risk of undergoing an acute or chronic rejection and therefore of loosing the allographt. This people can be treated with more potent immuosuppressive therapy to avoid rejection.

On the basis of above reported considerations, it is evident that the presence in blood of IP-10 levels higher than those of healthy subjects it is an useful index to point out the rejection probability of an allographt. Consequently, the IP-10 levels represents a new diagnostic method for such purpose.

## Claims

1. An in vitro diagnostic method for prevision of kidney transplantation rejection in which the blood levels of IP-10 of patients before transplantation are compared to the mean levels of healthy subjects and wherein an elevated pre-transplantation level of IP-10 can recognise the people with higher risk of undergoing an acute or chronic rejection and therefore of loosing the allograph.

## Patentansprüche

1. Diagnostisches in vitro Verfahren zur Vorhersage von Nierentransplantationsabstoßung, bei dem die Blutspiegel von IP-10 von Patienten vor der Transplantation mit den Durchschnittsspiegeln von gesunden Personen verglichen werden, und, bei dem ein erhöhter Prätransplantationsspiegel von IP-10 die Personen mit einem höheren Risiko, eine akute oder chronische Abstoßung zu unterlaufen und daher das Allotransplantat zu verlieren, erkennen kann.

## Revendications

1. Méthode diagnostique in vitro pour la prévision d'un rejet de greffe de rein, dans laquelle les taux sanguins de l'IP-10 des patients avant la transplantation sont comparés aux taux moyens des sujets sains, et dans laquelle un taux élevé de l'IP-10 avant la transplantation peut révéler les gens ayant un risque plus important de subir un rejet chronique ou aigu et donc de perdre l'allogreffe.
